# EUROPEAN PATENT APPLICATION

(11) **EP 0 821 909 A2**
(43) Date of publication of application: **04.02.1998**
(21) Application number: 97305277.2
(22) Date of filing: 15.07.1997
(51) Int. Cl.: A61B 5/00

(54) **Medical device**

(30) Priority: 30.07.1996 GB 9615924
(71) Applicant: Knoll Limited, Castle Boulevard, Nottingham NG7 1FW (GB)
(72) Inventor: Lean, Michael, Glasgow, G31 2ER. (GB); Murray, Ian, Nottingham, NG1 7AR. (GB); Woolf, Andrew, Nottingham, NG4 2HF. (GB)
(74) Representative: Jones, Stephen Anthony

(57) **Abstract**

A medical device is in the form of a tape (5) divided along its length into a plurality of discrete zones. A first zone (A) corresponds to a range of waist circumferences considered to be associated with a low health risk and a second zone (C) corresponds to a range of waist circumferences considered to be associated with a high health risk.

Preferably, the first and second zones (A,C) are indicated by different colours, eg green and red.

The device according to the invention is simple to use and provides a rapid indication of a person's risk of ill health. As such, the device is useful in health promotion and can be used to raise awareness or urge action on weight reduction.

## Description

This invention relates to a medical device, in particular to a medical device of the nature of a tape measure which is of use in identifying people at health risk.

About half of all British adults have a body mass index (weight (kg)/(height(m)²)) of > 25, while almost 15% have an index of > 30, and these proportions are rising. Given the lack of success in the management of obesity and increasing associated health costs, greater emphasis on prevention is needed, particularly in young people, who often have little contact with health services. While many health professionals are now familiar with the acceptable range for body mass index (20-25), most members of the public cannot readily calculate their index to establish their own risk or need for weight management. Charts developed by the Health Education Authority are helpful but are still not understood by many. Height must be measured accurately as small errors in the denominator are exaggerated by squaring.

There is therefore a need for a simple device with which a person's risk of ill health arising from obesity can be rapidly and easily identified by a medical practitioner or by that person.

According to the invention, there is provided a medical device in the form of a tape or the like, the tape or the like being divided along its length into a plurality of discrete zones, a first zone corresponding to a range of waist circumferences considered to be associated with a low health risk and a second zone corresponding to a range of waist circumferences considered to be associated with a high health risk.

The device according to the invention is advantageous in that it is simple to use and provides a rapid indication of a person's risk of ill health. As such, the device is useful in health promotion and can be used to raise awareness or urge action on weight reduction.

Preferably, the first and second zones are indicated by different colours applied to the respective zones. Most preferably, the first zone is indicated by the colour green (this being readily recognized as indicating a low risk) and the second zone by the colour red (this being recognized as a danger signal). Preferably, a third zone is provided intermediate the first and second zones, the third zone indicating that a person is close to being clinically obese and the need for caution and action to prevent further weight gain. The third zone is preferably indicated by the colour amber so that the first, third and second zones are represented successively by the familiar "traffic light" sequence of colours.

The tape is preferably wound on a reel housed within a casing. The reel is preferably spring biassed for automatic rewinding of the tape, and is provided with, or acted upon by, a mechanism for preventing rewinding. Such a mechanism may be a ratchet mechanism. In an alternative arrangement, however, the mechanism comprises a braking plate biassed into frictional engagement with the reel. The plate is preferably releasable from the reel by mechanical means, eg by means of a push button which may be formed integrally with the plate and which may protrude through an opening in the casing.

The casing is preferably provided with means for engaging the free end of the tape such that the tape may be withdrawn from the casing and passed around the waist, the free end being then engaged with the casing and the mechanism released to tauten the tape. The zone visible at the point at which the tape emerges from the casing may then be determined by inspection, either when the tape is in position around the waist or after disengaging the free end of the tape from the casing and removing the tape from the user's waist.

The medical device is preferably produced in separate versions for male and female users, due to differences between men and women in the waist circumferences corresponding to increased level of health risk. Alternatively, the two sides of the tape may carry the two different sets of zones. It may also be desirable to produce different versions of the device for use by people of different races.

The invention will now be described in greater detail, by way of illustration only, with reference to the accompanying drawings, in which
Figure 1 is a plan view, partially cut away, of a first embodiment of a medical device according to the invention, with a tape partially withdrawn;
Figure 2 is a sectional view along the line II-II in Figure 1;
Figure 3 is a partial view of a section of a tape forming part of the device of Figure 1;
Figure 4 shows the sequence of operations involved in the use of the device of Figure 1;
Figure 5 is a view similar to Figure 1 of a second embodiment of a medical device according to the invention; and
Figure 6 is a sectional view along the line VI-VI in Figure 5.

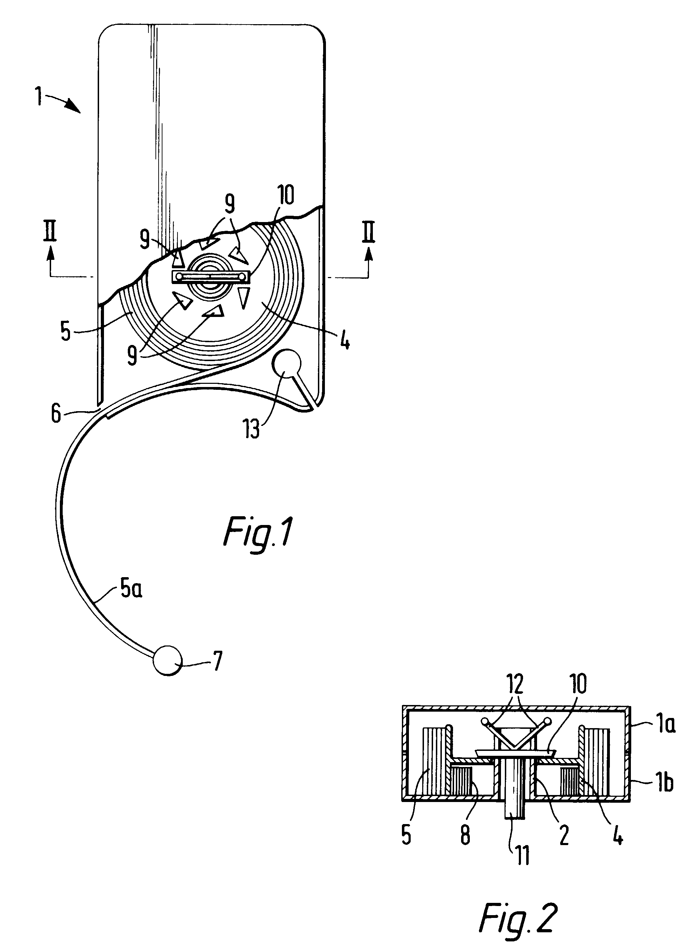

Referring first to Figure 1, a first embodiment of a medical device comprises a casing 1 formed from upper and lower halves 1a,1b (see Figure 2). An upstanding hollow boss 2 is formed integrally with the lower casing half 1b and a reel 4 is mounted upon it. A tape 5 is wound on the reel 4, the free end 5a of the tape 5 passing through a slot 6 at one corner of the casing 1. A cylindrical knob 7 is fixed to the free end 5a of the tape 5. The knob 7 prevents the tape 5 from being withdrawn entirely into the casing 1.

As can be seen in Figure 2, the reel 4 is of H-section and is biassed to a wound-up condition by a coiled ribbon spring 8. One end of the ribbon spring 8 is secured to the boss 2 and the other end to the reel 4.

Rewinding of the tape 5 on the reel 4 is prevented by ratchet formations 9 formed on the surface of the reel 4. The ratchet formations 9 engage a latch member 10 which is received in a channel formed in the upper end of the boss 2. The latch member 10 is formed integrally with a pin 11 which passes through the hollow boss 2 and protrudes from the underside of the casing 1. The ratchet can be released by depressing the protruding end of the pin 11 and raising the latch member 10 out of engagement with the ratchet formations 9. Two resilient limbs 12 are formed integrally with the latch member 10 and pin 11. The limbs 12 bear against the underside of the upper casing half 1a when the end of the pin 11 is pressed upwards and thus bias the pin 11 to the position shown in Figure 2.

The ratchet formations 9 are ramped so that the latch member 10 rides over them when the tape is unwound by the user drawing on the free end 5a.

A recess 13 is formed in the casing 1 and is dimensioned to receive the cylindrical knob 7 and the free end 5a of the tape 5.

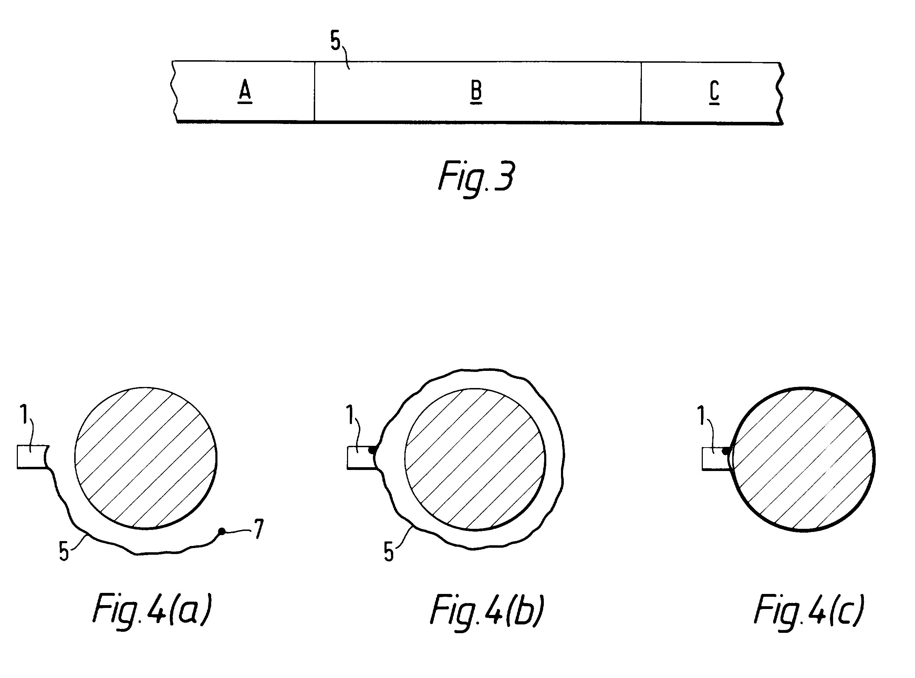

As the tape 5 is withdrawn from the casing 1, the first portion A of tape 5 is coloured green. The length of this portion A of the tape 5 corresponds to the range of waist circumferences considered to be associated with low health risk. The next, relatively short, portion B of tape 5 is coloured amber and corresponds to a range of waist circumferences which are indicative of a need to avoid additional weight gain. The third and final portion C of tape 5 to be withdrawn is coloured red and corresponds to a range of waist circumferences considered to be associated with a high health risk. The three portions A,B,C of the tape 5 are shown in Figure 3.

In use, the free end 5a of the tape 5 is withdrawn manually from the casing 1, against the action of the spring 8 and passed around the waist of a person (represented by the hatched circles in Figure 4) whose health risk is being assessed (see Figure 4a). Once the tape 5 has been passed right around the waist, the knob 7 is located in the recess 13 (Figure 4b). The end of the pin 11 is then depressed, releasing the latch member 10 from the ratchet formations 9 and causing the tape 5 to be rewound onto the reel 4. The tape 5 is thus drawn into tight engagement with the person's waist (see Figure 4c). The surface of the casing 1 between the slot 6 and the recess 13 is curved to facilitate abutment with the user's waist.

The tape 5 is then inspected to see which portion A,B,C is visible at the point at which the tape 5 emerges from the casing 1. If it is the green portion A which is visible then this is indicative of low health risk; if the red portion C is visible the health risk is considered high. If the tape 5 has been withdrawn to the amber region B, then the user is made aware of the need to avoid further weight gain.

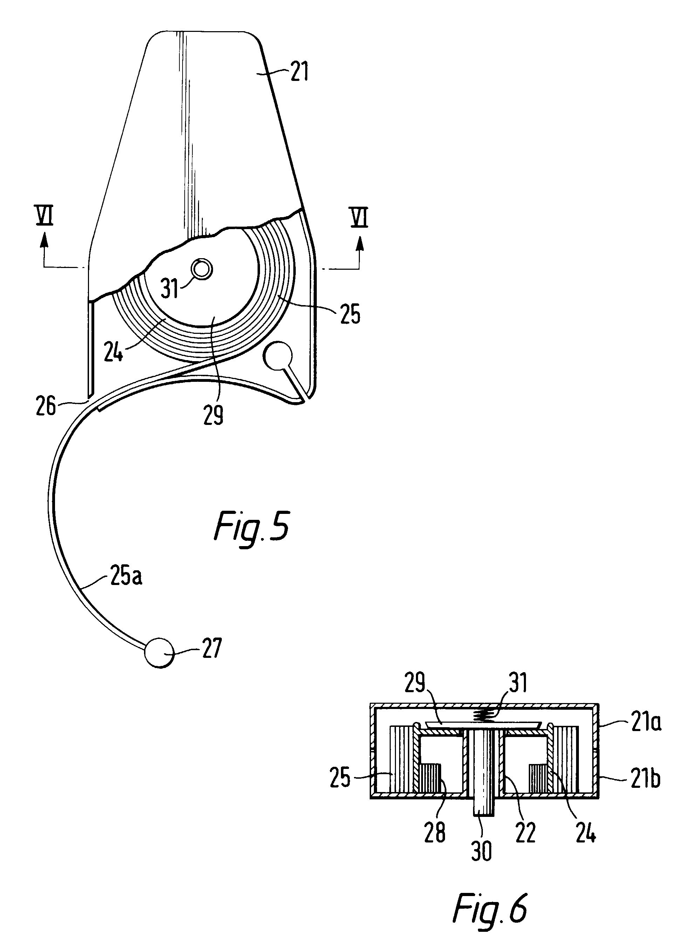

Referring now to Figures 5 and 6, a second embodiment of a medical device according to the invention comprises a casing 21 generally similar (though somewhat different in overall shape) to the casing 1 of the first embodiment. The casing 21 again comprises upper and lower halves 21a,21b. An upstanding hollow boss 22 is formed integrally with the lower casing half 21b and a reel 24 is mounted upon it. A tape 25 is wound on the reel 24, the free end 25a of the tape 25 passing through a slot 26 at one corner of the casing 21. A cylindrical knob 27 is fixed to the free end 25a of the tape 25.

As can be seen in Figure 6, the reel 24 is biassed to a wound-up condition by a coiled ribbon spring 28. One end of the ribbon spring 28 is attached to the boss 22 and the other end to the reel 24.

The second embodiment differs from the first in the nature of the mechanism by which rewinding of the tape 25 onto the reel 24 is prevented. Instead of a ratchet mechanism, the second embodiment has a braking plate 29 which is formed integrally with a pin 30. The pin 30 passes through the hollow boss 22 and protrudes from the underside of the casing 1. The braking plate 29 is urged into frictional engagement with the reel 24 by a compression spring 31, one end of which is seated in a blind bore in the pin 30 and the other end of which bears against the internal surface of the upper casing half 21a.

The tape 25 can be withdrawn from the casing 21, against the action of the ribbon spring 28 and the braking plate 29. Rewinding of the tape 25 onto the reel 24 is prevented by engagement of the braking plate 29 with the reel 24. The braking plate 29 can be released from the reel 24 by pressing the end of the pin 30, whereupon the tape 25 rewinds under the action of the ribbon spring 28.

The tape 25 is divided into three portions, as shown in Figure 3 and is used in the same manner as the first embodiment, ie as shown in Figure 4.

## Claims

1. A medical device in the form of a tape (5) or the like, the tape (5) or the like being divided along its length into a plurality of discrete zones (A,B,C), a first zone (A) corresponding to a range of waist circumferences considered to be associated with a low health risk and a second zone (C) corresponding to a range of waist circumferences considered to be associated with a high health risk.

2. A device as claimed in Claim 1, wherein the first and second zones (A,C) are indicated by different colours applied to the respective zones.

3. A device as claimed in Claim 2, wherein the first zone (A) is indicated by the colour green and the second zone (C) by the colour red.

4. A device as claimed in any preceding claim, wherein a third zone (B) is provided intermediate the first and second zones (A,C).

5. A device as claimed in any preceding claim, wherein the tape (5) is wound on a reel (4;24) housed within a casing (1).

6. A device as claimed in Claim 5, wherein the reel (4;24) is spring biassed for automatic rewinding of the tape (5), and is provided with, or acted upon by, a mechanism for preventing rewinding.

7. A device as claimed in Claim 6, wherein the mechanism is a ratchet mechanism (9,10).

8. A device as claimed in Claim 6, wherein the mechanism comprises a braking plate (29) biassed into frictional engagement with the reel (24), the plate (29) being releasable from the reel (24) by means of a push button (11) which protrudes through an opening in the casing (1).

9. A device as claimed in Claim 5, wherein the casing (1) is provided with means (13) for engaging the free end of the tape (5) such that the tape (5) may be withdrawn from the casing (1) and passed around the waist, the free end being then engaged with the casing (1) and the mechanism released to tauten the tape (5).
